# EUROPÄISCHE PATENTSCHRIFT

(11) **EP 2 773 406 B1**
(45) Veröffentlichungstag und Bekanntmachung des Hinweises auf die Patenterteilung: **14.09.2016**
(21) Anmeldenummer: 12797737.9
(22) Anmeldetag: 05.11.2012
(51) Int. Cl.: A61M 15/00, A61M 11/00, A61M 15/08

(54) **EINWEGDOSIERER**
SINGLE-USE DOSING DEVICE
DOSEUR À USAGE UNIQUE

(30) Priorität: 04.11.2011 DE 102011055074; 17.07.2012 DE 102012106438
(43) Veröffentlichungstag der Anmeldung: 10.09.2014
(73) Patentinhaber: F + K Innovationen GmbH & Co.KG, 76543 Baden-Baden (DE)
(72) Erfinder: Fusch, Karl-Heinz, 78315 Radolfzell (DE); Umsonst-Kübler, Petra, 76534 Baden-Baden (DE)
(74) Vertreter: Patentanwälte und Rechtsanwalt Weiß, Arat & Partner mbB
(86) Internationale Anmeldenummer: PCT/EP2012/071777
(87) Internationale Veröffentlichungsnummer: WO 2013/064677

(56) Entgegenhaltungen:
- EP-A1- 1 449 595
- WO-A1-2004/022244
- DE-A1- 19 905 993
- US-A- 4 623 337
- US-B1- 6 401 987

## Beschreibung

Die Erfindung betrifft einen Einwegdosierer nach dem Oberbegriff des Anspruchs 1.

### Stand der Technik

Aus dem Stand der Technik sind verschiedene Einwegdosierer bekannt, welche aber sehr materialaufwendig sind und entsprechend viel Müll verursachen.

In diesem Zusammenhang wird auf DE 199 05 993 A1 offenbart einen Spender für fliessfähige Medien, bei dem eine Pumpenkammer und ihr Verschluss austauschbar sind. Weiter wird auf die WO 2004/022244 A1 hingewiesen, welche einen Apparat zum Ausbringen von Flüssigkeiten, eine dazu passende Behälterpatrone zur Bevorratung der Flüssigkeit und das Ensemble aus beidem offenbart.

Daneben wird auf die US 6,401,987 B1 hingewiesen, welche eine weitere aus dem Stand der Technik bekannte Ausbringvorrichtung für ein flüssiges Medium aufzeigt. Ausserdem wird auf die EP 1 449 595 A1 hingewiesen, welche einen Spender zum Abgeben eines flüssigen oder pastösen Mediums offenbart, bei dem über eine Federkonstruktion ein Ausbringelement nahc dem Betätigen wieder zurückgestellt wird.

### Aufgabe

Aufgabe der Erfindung ist es einen Einwegdosierer zur Verfügung zu stellen, welcher die Nachteile aus dem Stand der Technik weitestgehend abstellt und einen Benutzerfreundlichen Aufbau aufweist. Hierzu soll insbesondere erreicht werden, dass unnötiger Müll verursacht wird. Die Anwendung des erfindungsgemässen Einwegdosierers soll fehlerfrei auch für ungeschultes Personal möglich sein.

### Lösung der Aufgabe

Die Aufgabe wird durch die Merkmale des Anspruchs 1 gelöst.

Bei dem erfindungsgemässen Einwegdosierer handelt es sich um einen Einwegdosierer für nasale Spray- oder Tropfenanwendungen. Mit einer Ampulle und einem Betätigungsadapter. Allerdings ist der erfindungsgemässe Einwegdosierer zweiteilig aufgebaut. D. h. in erster Linie, dass die Ampulle und der Betätigungsadapter trennbar von einander ausgebildet sind. Die Koppelung der Ampulle mit dem Betätigungsadapter ist derart gestaltet, dass der Betätigungsadapter von einer beispielsweisen entleerten Ampulle abnehmbar und an einer anderen Ampulle wieder verwendbar ist oder auch auf die ursprüngliche Ampulle ankoppelbar ist.

In diesem Zusammenhang spielt der Inhalt der Ampulle eine untergeordnete Rolle. Regelmässig wird sich dabei um einen irgendwie gearteten Wirkstoff oder eine Salzlösung handeln, welche nasal angewandt wird. Denkbar ist auch, dass die Ampulle aus unterschiedlichen Materialien oder Glas hergestellt ist.

Der erfindungsgemässe Einwegdosierer ist weiterhin derart ausgebildet, dass der Betätigungsadapter ein Führungselement zur Betätigung eines Ausklinkimpulses des flüssigen Wirkstoffs der Ampulle aufweist. Das Führungselement ist dabei halbkreisförmig ausgebildet. In einem bevorzugten Ausführungsbeispiel schliesst an das Führungselement ein Betätigungselement an, welches jeweils endseitig an dem Führungselement in etwa rechtwinklig abragt.

Der erfindungsgemässe Einwegdosierer ist derart gestaltet, dass die Ampulle mit dem Betätigungsadapter über einen Bajonettverschluss verbindbar ist. Ausserdem ist der Betätigungsadapter derart ausgebildet, dass das Führungselement mit einem Federelement über ein Betätigungselement in Wirkverbindung steht. In der Regel ist das Betätigungselement entweder zwischen dem Führungselement und dem Federelement angeordnet oder das Betätigungselement ist zwischen dem Führungselement und einer Aufnahme für eine Ampulle angeordnet, wobei eine erfindungsgemässe Aufnahme derart gestaltet ist, dass sie einen Bajonettverschluss im Inneren aufweist, in die wiederum ein Nocken einer erfindungsgemässen Ampulle eingedreht werden kann. Somit ist der erfindungsgemässe Einwegdosierer so angeordnet, dass das Federelement mit dem Kolben in Wirkverbindung steht.

Ausserdem wird eine Ampulle für nasale Spray- oder Tropfenanwendungen mit einem flüssigen Wirkstoff derart angeordnet, dass die Ampulle einen Nocken zur lösbaren Verbindung mit dem Bajonettverschluss des Betätigungsadapters aufweist. Weiterhin weist eine erfindungsgemässe Ampulle auch einen entfernbaren Produktschutz auf. Dieser Produktschutz ist derart gestaltet, dass er durch eine Drehbewegung entfernt werden kann. Dadurch wird wiederum eine Ausbringöffnung der Ampulle freigegeben. In einem anderen Ausführungsbeispiel ist der Produktschutz derart mit einem Kunststoffelement im Inneren der Ampulle verbunden, dass das Kunststoffelement anschliessend nach dem Abdrehen des entfernbaren Produktschutzes eine Sprayfunktion bzw. eine Tropfenfunktion der Ampulle unterstützt bzw. erfüllt. Weiter weist die erfindungsgemässe Ampulle einen im Inneren der Ampulle verschiebar angeordneten Stopfen auf. Dieser Stopfen kann im Zusammenspiel mit dem Betätigungsadapter insbesondere dem Kolben des Betätigungsadapters, welcher mit dem Stopfen in Rückverbindung steht zur Ausbringöffnung hin gedrückt werden. Dabei wird der flüssige Wirkstoff durch den Stopfen an dem Kunststoffelement vorbeigeleitet und durch die Kunststofföffnung nach aussen befördert.

### Figurenbeschreibung

Weitere Vorteile, Merkmale und Einzelheiten der Erfindung ergeben sich aus der nachfolgenden beschreibung bevorzugter Ausführungsbeispiele sowie anhand der Zeichnungen. Diese zeigen in
Figur 1 einen Querschnitt eines erfindungsgemässen Bestätigungsadapters sowie eine Glas- und/oder Kunststoffampulle, jeweils in einem ausgebauten Zustand;
Figur 2 einen Querschnitt eines erfindungsgemässen Betätigungsadapters mit einer Glasampulle in einem eingebauten Zustand.

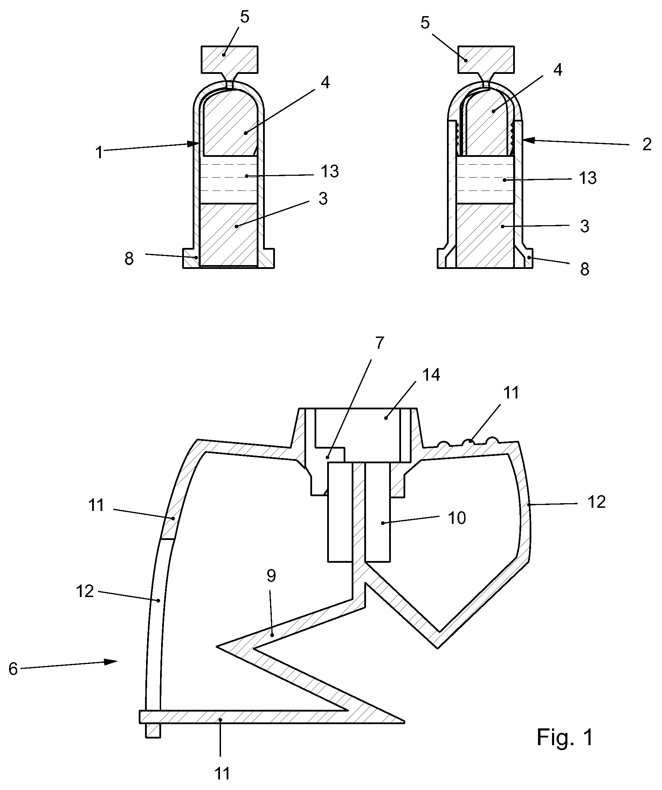

Gemass Figur 1 weist ein Ausführungsbeispiel einer erfindungsgemässen Vorrichtung eine Glas- bzw. eine Kunststoffampulle 1, 2 auf. Diese Ampulle 1, 2 verfügt an einem Ende über einen Produktschutz 5 und an einem entgegengsetzen Ende zumindest über einen Nocken 8, wobei dieser zumindest einen Nocken 8 Teil eines Bajonettverschlusses 7 ist. Im Inneren der Ampulle 1, 2 befindet sich an einem dem Produktschutz 5 entgegengesetztem Ende ein Stopfen 3. Im betriebsbereiten Zustand befindet sich im Inneren der Ampulle 1, 2 weiterhin eine Wirkstofflösung 13. Ausserdem befindet sich im Inneren der Ampulle 1, 2 ein Kunststoffelement 4.

Ein Betätigungsadapter 6 weist gemäss Figur 1 zumindest ein Betätigungselement 11, einen Kolben 10, einen Teil eines Bajonettverschlusses 7, eine Aufnahme 14, ein Führungs- und Betätigungselement 12 sowie ein Federelement 9 auf. Die Aufnahme 14 ist derart gestaltet, dass im Inneren ein Bajonettverschluss 7 vorhanden ist, welche mit dem Nocken 8 der Ampulle 1, 2 zusammenwirkt. Die Ampulle 1, 2 kann mit Hilfe des Nockens 8 und des Bajonettverschlusses 7 in der Aufnahme 14 lösbar eingedreht bzw. eingeklipst werden. Die Aufnahme 14 wiederum umfasst einen Kolben 10, welcher mit dem Stopfen 3 in Wirkverbindung steht. D. h. bei der Bewegung des Kolbens 10 hin

### Figurenbeschreibung

Weitere Vorteile, Merkmale und Einzelheiten der Erfindung ergeben sich aus der nachfolgenden beschreibung bevorzugter Ausführungsbeispiele sowie anhand der Zeichnungen. Diese zeigen in
Figur 1 einen Querschnitt eines erfindungsgemässen Bestätigungsadapters sowie eine Glas- und/oder Kunststoffampulle, jeweils in einem ausgebauten Zustand;

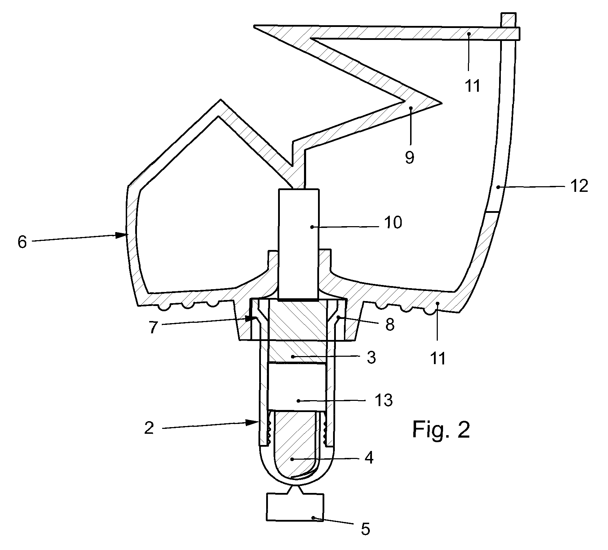

Figur 2 einen Querschnitt eines erfindungsgemässen Betätigungsadapters mit einer Glasampulle in einem eingebauten Zustand.

Gemass Figur 1 weist ein Ausführungsbeispiel einer erfindungsgemässen Vorrichtung eine Glas- bzw. eine Kunststoffampulle 1, 2 auf. Diese Ampulle 1, 2 verfügt an einem Ende über einen Produktschutz 5 und an einem entgegengsetzen Ende zumindest über einen Nocken 8, wobei dieser zumindest einen Nocken 8 Teil eines Bajonettverschlusses 7 ist. Im Inneren der Ampulle 1, 2 befindet sich ein dem Produktschutz 5 entgegengesetztem Ende ein Stopfen 3. Im betriebsbereiten Zustand befindet sich im Inneren der Ampulle 1, 2 weiterhine eine Wirkstofflösung 13. Ausserdem befindet sich im Inneren der Ampulle 1, 2 ein Kunststoffelement 4.

Ein Betätigungsadapter 6 weist gemäss Figur 1 zumindest ein Betätigungselement 11, einen Kolben 10, einen Teil eines Bajonettverschlusses 7, eine Aufnahme 14, ein Führungs- und Betätigungselement 12 sowie ein Federelement 9 auf. Die Aufnahme 14 ist derart gestaltet, dass im Inneren ein Bajonettverschluss 7 vorhanden ist, welche mit dem Nocken 8 der Ampulle 1, 2 zusammenwirkt. Die Ampulle 1, 2 kann mit Hilfe des Nockens 8 und des Bajonettverschlusses 7 in der Aufnahme 14 lösbar eingedreht bzw. eingeklipst werden. Die Aufnahme 14 wiederum umfasst einen Kolben 10, welcher mit dem Stopfen 3 in Wirkverbindung steht. D. h. bei der Bewegung des Kolbens 10 hin zu der Ampulle 1, 2 wird der Stopfen 3 im Inneren der Ampulle 1, 2 hin zu dem Produktschutz 5 geschoben. Wenn der Produktschutz 5 dann abgenommen ist, wird dabei durch den Stopfen 3 der flüssigen Wirkstoff 13 an dem Kunststoffelement 4 vorbei durch die vorhandene Öffnung gedrück. Der Kolben 10 wiederum umfasst ein Federelement 9, welches mit dem Betätigungselement 11 einstückig verbunden ist. Das Betätigungselement 11 wiederum ist in eine in dem Führungs- und Betätigungselement 12 verschiebbar gelagert. D. h., wenn das Betätigungselement 11 zum einen als Gegenlager gehalten wird und zum anderen zum Drücken des Federelements 9 hin zu der Aufnahme 14 genutzt wird, gibt das Führungs- und Betätigungselement 12 vor, inwieweit dieses Drücken von Statten gehen kann. Das Federelement 9 wiederum gewährleistet eine Rückstellkraft, die dann den Kolben 10 bei nicht mehr notwendigem Gebrauch zurück in die Ausgangsposition zieht, um die dann entleerte Ampulle 1, 2 aus der Aufnahme 14 zu entfernen.

In Figur 2 ist eine Vorrichtung aus der Ampulle 2 und dem Betätigungsadapter 6 im eingebauten Zustand gezeigt, wobei an dieser Stelle darauf hingewiesen sei, dass die Verwendung der Ampulle 2 aus Glas hier nur beispielhaft zu verstehen ist, die Verwendung einer Ampulle 1 aus Kunststoff ist ebenso denkbar. Weiterhin sei darauf hingewiesen, dass die in Figur 1 genannten Bezugsziffern und ihnen entsprechende Elemente der Vorrichtung sinngemäss auch für Figur 2 gelten sollen.

In Figur 2 ist zu erkennen, wie die mittels des Bajonettverschlusses 7 in den Betätigungsadapter 6 arritierte Ampulle 2 mit dem Betätigungsadapter 6 zusammen wirkt. Die Funktionsweise der vorliegenden Erfindung ist folgende:
Der in diesem Ausführungsbeispiel einstückig geformte Betätigungsadapter 6 wird bei dessen Montage vorgespannt, in dem der Kolben 10 in eine korrespondierende Aufnahme 14 eingebracht wird und um Ende eines Griffelementes 11 mit einem Führungs- und Betätigungselement 12 in Eingriff gebracht wird. Der Betätigungsadapter 6 wird, wie in Figur 2 dargestellt, mit der Ampulle 2 geladen. Wird der Produktschutz 5 entfernt, so kann mittels eines vorzugsweise durch mehrere Finger eines Benutzers auf die Betätigungsflächen 11 ausgeübten Druckes der Kolben 10 und somit auch der Stopfen 3 in Richtung eines dem Stopfen 3 entgegengesetzten Endes der Ampulle 2 bewegt werden. Dies führt nun zu einem Austritt der Wirkstofflösung 13 aus diesem Ende, wobei mittels des Kunststoffelementes 4 Tropfen oder ein Spray erzeugt werden.

Nach einer Anwendung muss nur die Ampulle 2 entsorgt werden, der Betätigungsadapter 6 kann mehrfach verwendet werden.

### Bezugszeichenliste

1. Kunststoffampule
2. Glasampulle
3. Stopfen
4. Kunststoffelement
5. Produktschutz
6. Betätigungsadapter
7. Bajonettverschluss
8. Nocke
9. Federelement
10. Kolben
11. Betätigungselement
12. Führungs- und Betätigungselement
13. Wirkstofflösung

## Patentansprüche

1. Einwegdosierer für nasale Spray- oder Tropfenanwendungen mit einem Lagerelement in Form einer Ampulle und einem Ausbringelement in Form eines Betätigungsadapters (6), wobei die Ampulle (1, 2) lösbar mit dem Betätigungsadapter (6) verbunden ist, wobei der Betätigungsadapter (6) ein Führungs- und Betätigungselement (12) zur Betätigung eines Ausbringimpulses eines flüssigen Wirkstoffs (13) aus der Ampulle (1, 2) aufweist, wobei der Betätigungsadapter (6) ein Betätigungselement (11), einen Kolben (10), eine Aufnahme (14), ein Führungs- und Betätigungselement (12) sowie ein Federelement (9) aufweist, wobei das Führungs- und Betätigungselement (12) mit einem Federelement (9) über ein Betätigungselement (11) in Wirkverbindung steht, wobei das Federelement (9) mit einem Kolben (10) in Wirkverbindung steht, wobei die Ampulle (1, 2) mit dem Betätigungsadapter (6) über einen Bajonettverschluss (7) verbindbar ist, wobei die Ampulle (1, 2) einen Nocken (8) zur lösbaren Verbindung mit dem Bajonettverschluss (7) vorhanden ist, wobei an der Ampulle (1, 2) ein entfernbarer Produktschutz (5) vorhanden ist und in der Ampulle (1, 2) ein Kunststoffelement (4) angeordnet ist und die Ampulle (1, 2) einen im Inneren der Ampulle (1, 2) verschiebbar angeordneten Stopfen (3) aufweist, **dadurch gekennzeichnet, dass** die Ampulle (1, 2) an einem Ende über einen Produktschutz (5) und an einem entgegengsetzen Ende zumindest über den Nocken (8) verfügt und im Inneren der Ampulle (1, 2) befindet sich an dem dem Produktschutz (5) entgegengesetzten Ende der Stopfen (3), wobei die Aufnahme (14) den Kolben (10) umfasst, welcher mit dem Stopfen (3) in Wirkverbindung steht, wobei bei der Bewegung des Kolbens (10) hin zu der Ampulle (1, 2) der Stopfen (3) im Inneren der Ampulle (1, 2) hin zu dem Produktschutz (5) geschoben wird, wobei der Produktschutz (5) durch eine Drehbewegung entfernbar ist.

## Claims

1. A single-use dosing device for nasal spray or drop applications, with a bearing element in the form of an ampoule and a discharge element in the form of an actuating adapter (6), wherein the ampoule (1, 2) is connected detachably to the actuating adapter (6), wherein the actuating adapter (6) has a guide and actuation element (12) for actuating a pulse for discharging a liquid active substance (13) from the ampoule (1, 2), wherein the actuating adapter (6) has an actuation element (11), a piston (10), a receptacle (14), a guide and actuation element (12) and also a spring element (9), wherein the guide and actuation element (12) is in an operative connection with a spring element (9) via an actuation element (11), wherein the spring element (9) is in an operative connection with a piston (10),
wherein
the ampoule (1, 2) can be connected to the actuating adapter (6) via a bayonet lock (7), wherein the ampoule (1, 2) a cam (8) for detachably connecting to the bayonet lock (7) is present, wherein a removable product protection means (5) is present on the ampoule (1,2) and a plastics-material element (4) is arranged in the ampoule (1, 2) and the ampoule (1, 2) has a stopper (3) arranged displaceably in the interior of the ampoule (1, 2), **characterised in that** the ampoule (1, 2) has at one end a product protection means (5) and at an opposite end at least the cam (8), and in the interior of the ampoule (1, 2) there is on the end opposite the product protection means (5) the stopper (3), the receptacle (14) comprising the piston (10), which is in an operative connection with the stopper (3), with upon the movement of the piston (10) towards the ampoule (1, 2) the stopper (3) being pushed in the interior of the ampoule (1, 2) towards the product protection means (5), the product protection means (5) being able to be removed by a rotary movement.

## Revendications

1. Doseur à usage unique pour applications par vaporisation ou en gouttes avec un élément de support sous forme d'une ampoule et un élément de distribution sous forme d'un adaptateur d'actionnement (6), dans lequel l'ampoule (1, 2) est connectée de manière amovible à l'adaptateur d'actionnement (6), dans lequel l'adaptateur d'actionnement (6) présente un élément de guidage et d'actionnement (12) destiné à actionner une impulsion de sortie d'un substance active liquide (13) de l'ampoule (1, 2), dans lequel l'adaptateur d'actionnement (6) présente un élément d'actionnement (11), un piston (10), un réceptacle (14), un élément de guidage et d'actionnement (12) ainsi qu'un élément de ressort (9), dans lequel l'élément de guidage et d'actionnement (12) est en liaison active avec un élément de ressort (9) par l'intermédiaire d'un élément d'actionnement (11), l'élément de ressort (9) étant en liaison active avec un piston (10), dans lequel l'ampoule (1, 2) peut être connectée à l'adaptateur d'actionnement (6) par l'intermédiaire d'une fermeture à baïonnette (7), dans lequel à l'ampoule (1, 2) est présente une came (8) pour une connexion amovible avec la fermeture à baïonnette (7), dans lequel à l'ampoule (1, 2) est présente une protection de produit amovible (5) et dans l'ampoule (1, 2) est disposé un élément en matière plastique (4), et l'ampoule (1, 2) présente un bouchon déplaçable (3) disposé à l'intérieur de l'ampoule (1, 2), **caractérisé par le fait que**
l'ampoule (1, 2) dispose à une extrémité d'une protection de produit (5) et à une extrémité opposée au moins d'une came (8) et qu'à l'intérieur de l'ampoule (1, 2) se trouve, à l'extrémité opposée à la protection de produit (5), le bouchon (3), dans lequel le réceptacle (14) comporte le piston (10) qui est en liaison active avec le bouchon (3), dans lequel, lors du déplacement du piston (10) vers l'ampoule (1, 2), le bouchon (3) est poussé à l'intérieur de l'ampoule (1, 2) vers la protection de produit (5), dans lequel la protection de produit (5) est amovible par un mouvement de rotation.
